**Europäisches Patentamt**

**European Patent Office**

**Office européen des brevets**

(19)

(11) Veröffentlichungsnummer: **0 109 460**

**A1**

(12) # EUROPÄISCHE PATENTANMELDUNG

(21) Anmeldenummer: **82110830.5**

(22) Anmeldetag: **23.11.82**

(51) Int. Cl.³: **A 61 M 1/00**

(43) Veröffentlichungstag der Anmeldung:
**30.05.84 Patentblatt 84/22**

(84) Benannte Vertragsstaaten:
**AT BE CH DE FR GB IT LI LU NL SE**

(71) Anmelder: **Sterimed Gesellschaft für medizinischen Bedarf mbH**
**Fasanerieweg 15**
**D-6600 Saarbrücken(DE)**

(72) Erfinder: **Härle, Anton, Dr.**
**Drechslerweg 40**
**D-4400 Münster(DE)**

(74) Vertreter: **Suchy, Herbert, Dr.**
**Byk Gulden Lomberg Chemische Fabrik GmbH**
**Byk-Gulden-Strasse 2 Postfach 6500**
**D-7750 Konstanz(DE)**

(54) **Sauggerät zur Aufnahme und Abgabe, z.B. Entsorgung von medizinischen Sekreten oder Flüssigkeiten.**

(57) Sauggerät zum Sammeln und Entsorgen von Sekreten und anderen medizinischen Flüssigkeiten umfassend einen Saugbehälter (1, 1a). einen in den Saugbehälter (1, 1a) einhängbaren flexiblen Auffangbeutel (6, 6a), vorzugsweise einen Folienbeutel, der an seiner Einfüllöffnung abdichtend und fest im Saugbehälter (1, 1a) gehalten ist, wobei der Auffangbeutel (6, 6a) einen Anschluß zu einer Vakuumquelle und einen Anschluß für eine Absaugleitung aufweist, eine unabhängig vom Auffangbeutel (6, 6a) zwischen der Innenseite des Saugbehälters (1, 1a) und der Außenseite des Auffangbeutels (6, 6a) vorgesehene Anschlußöffnung für eine zusätzliche Saugleitung (3), und einen auf die Außenseite des Auffangbeutels (6, 6a) wirkenden Druckanschluß.

EP 0 109 460 A1

./...

Fig.1

0109460

**Sauggerät zur Aufnahme und Abgabe, z.B. Entsorgung von medizinischen Sekreten oder Flüssigkeiten**

## Technisches Gebiet

Die Erfindung bezieht sich auf ein Sauggerät mit flexiblem Auffangbeutel zum Ansaugen und Sammeln von Sekreten und anderen medizinischen Flüssigkeiten sowie zur Entsorgung dieser angesaugten Stoffe unter keimfreien Bedingungen.

## Stand der Technik

Das Sammeln und Entsorgen von medizinischen Flüssigkeiten im Krankenhaus, wie z.B. Urin, Wundsekrete und Sekrete bei Operationen, bringen erhebliche Kontaminationsrisiken mit sich. Beispielsweise werden Urinbeutel entweder in einem Entsorgungsraum ausgeleert und verworfen, oder sie verbleiben beim Patienten und werden über einen Ablaßhahn in ein Auffanggefäß entleert. Beide Vorgangsweisen kontaminieren zwangsläufig die Umgebungsluft und das Pflegepersonal. Entsprechendes gilt für Sekretflaschen zum Auffangen von Wundsekreten, wie sie in der DE-AS 26 39 715 offenbart sind, sowie bei den sogenannten Operationssaugern, die zum Auffangen größerer Sekretmengen bei Operationen verwendet werden. Bei diesen Operationssaugern ist es üblich, verhältnismäßig große Auffangbehälter, beispielsweise aus Glas, auf fahrbaren Traggestellen anzuordnen. Sind diese Saugbehälter gefüllt, wird die gesamte Saugvorrichtung an einen Reservebehälter angeschlossen und der volle Behälter wird durch einfaches Dekantieren entleert.

Aus der DE-PS 21 06 631 ist eine Sekretsaugflasche bekannt, in die ein flexibler Auffangbeutel eingehängt werden kann. In den Auffangbeutel münden zwei Leitungen, wovon die eine zum Patienten führt und die andere mit einer Unterdruck erzeugenden Vorrichtung verbunden ist. Der Zwischenraum zwischen dem Auffangbeutel und der Sekretflasche ist ebenfalls mit der Unterdruck erzeugenden Vorrichtung verbunden. Sobald der Auffangbeutel gefüllt ist, oder die Sekretabsaugung aus anderen Gründen beendet wird, werden der Auffangbeutel und der Zwischenraum zwischen Auffangbeutel und Sekretsaugflasche belüftet. Der Auffangbeutel wird dann samt Inhalt aus der Sekretsaugflasche entnommen und, gewünschtenfalls nach vorheriger Entleerung, verworfen. Nach diesem Prinzip arbeitende Sekretsaugflaschen mit Auffangbeutel sind weiterhin aus den US-PSen 3 719 197, 3 780 738 und 4 060 107 sowie als weit verbreitete Handelsprodukte bekannt. Auch beim Wechseln der Auffangbeutel dieser Sekretsaugflaschen nach dem Stand der Technik entstehen Kontaminationsprobleme, da der Beutelinhalt und die Saugleitung beim Abnehmen des Beutels mit der Umgebungsluft in Kontakt kommen. Beim Entleeren dieser Beutel treten die gleichen Probleme wie beim Entleeren von Urinbeuteln auf.

Auf dem Gebiet der Bluttransfusion ist es aus den US-PSen 2 597 715 und 3 507 278 bekannt, die Überführung von Blut aus einem flexiblen Blutbeutel, der sich in einem starren Behälter befindet, in das Gefäßsystem eines Patienten durch Anwendung von Gasdruck zu unterstützen.

Beschreibung der Erfindung

Der vorliegenden Erfindung liegt die Aufgabe zugrunde, ein Sauggerät mit flexiblem Auffangbeutel zu schaffen, mit dem medizinische Flüssigkeiten in einem geschlossenen System ohne Kontakt mit der Umgebungsluft gesammelt und entsorgt werden können.

Die Lösung der der Erfindung zugrundeliegenden Aufgabe besteht nun darin, daß an einer Saugvorrichtung bestehend aus einem Außenbehälter und einem darin angeordneten flexiblen Auffangbeutel, zusätzlich ein Druckanschluß vorgesehen wird, der lediglich auf die Außenseite des Auffangbeutels einwirkt und der es nun ermöglicht, ohne daß ein Eröffnen des Auffangbeutels notwendig ist, für eine Entleerung des Inhaltes des Auffangbeutels Sorge zu tragen.

Gegenstand der Erfindung ist ein Sauggerät zum Sammeln und Entsorgen von Sekreten und anderen medizinischen Flüssigkeiten umfassend einen Saugbehälter, einen in den Saugbehälter einhängbaren flexiblen Auf-

0109460

fangbeutel, vorzugsweise einen Folienbeutel, der an seiner Einfüllöffnung abdichtend und fest im Saugbehälter gehalten ist, wobei der
Auffangbeutel einen Anschluß zu einer Vakuumquelle und einen Anschluß
für eine Absaugleitung aufweist, eine unabhängig vom Auffangbeutel
zwischen der Innenseite des Saugbehälters und der Außenseite des Auffangbeutels vorgesehene Anschlußöffnung für eine zusätzliche Saugleitung, und einen auf die Außenseite des Auffangbeutels wirkenden
Druckanschluß.

Weitere Gegenstände der Erfindung ergeben sich aus den Patentansprüchen.

Die Erfindung schlägt zwei in Abhängigkeit vom Anwendungsbereich gestaltete Einrichtungen vor, wobei einmal eine Ansauganlage vorgesehen
wird, mit der medizinische Flüssigkeiten in einen Auffangbeutel gesaugt werden können, so daß diese Einrichtung wie handelsübliche
Operationssauger arbeitet, wobei aber das Entleeren des Auffangbeutels durch Einstellen eines Überdruckes im Raum zwischen dem
Saugbehälter und dem flexiblen Auffangbeutel des Sauggeräts erfolgt,
so daß die Abgabe der aufgefangenen Flüssigkeit unabhängig von ihrer
Menge ohne Berührung mit der Umgebungsluft erfolgen kann. Eine Kontaminierung der Bedienungspersonen und der aufgefangenen Flüssigkeit (um
beispielsweise chemische Untersuchungen störungsfrei durchführen zu
können) wird dadurch vermieden.

Es ist nunmehr möglich, beispielsweise alle Urinbeutel oder Sekretauffangbehälter der einzelnen Patienten einer Pflegestation unter sterilen Bedingungen an Ort und Stelle zu leeren. Hierzu ist die Absaugleitung des Sauggerätes mit einer Punktiernadel ausgestattet, womit die
Urinbeutel oder Sekretauffangbehälter über deren Punktierzentrum entleert werden (Sekretsaugflaschen mit Punktierzentrum sind z.B. in der
DE-AS 28 20 517 beschrieben). Sind nunmehr mehrere oder alle Urinbeutel oder Sekretauffangbehälter einer Pflegestation abgesaugt, wird
das erfindungsgemäße Sauggerät in den Entsorgungsraum gebracht und durch
Anwendung von Druck auf den Zwischenraum zwischen dem Saugbehälter und dem flexiblen Auffangbehälter entleert. Die zu entleerende Flüssigkeit kann direkt in die Entsorgungsleitung des Krankenhauses gegeben werden, so daß die Bedienungsperson mit dem Inhalt des flexiblen Auffangbeutels nicht in Kontakt kommt.

Mit dem erfindungsgemäßen Gerät wird also sowohl die Infektionsgefahr erheblich verringert und gleichzeitig das Entleeren der Urinbeutel oder Sekretauffangbehälter erheblich beschleunigt.

Weiterhin wird die übliche vom Patienten tragbare Saugflasche dahingehend geändert, daß in diese Saugflasche ein zusätzlicher Auffangbeutel eingebracht wird, wobei durch Erzeugen eines Unterdruckes an der Außenseite des Auffangbeutels nunmehr die Saugflasche arbeitsbereit ist, andererseits durch Erzeugen eines Überdrucks an der Außenseite des Auffangbeutels der Inhalt des Auffangbeutels ohne Luftberührung abgegeben werden kann, so daß die aufgefangene Flüssigkeit, beispielsweise Wundsekret, weiteren Untersuchungen, aber auch insbesondere weiteren Verarbeitungen, beispielsweise zur Gewinnung von Albumin, oder der neuerdings an Bedeutung gewinnenden Isolierung von Leukozyten, zugeführt werden kann.

Über die vorstehend erläuterten Vorteile durch den Einsatz der erfindungsgemäßen Einrichtungen hinaus wird bewirkt, daß ein relativ kostengünstig herstellbarer Auffangbeutel geschaffen wird, der nach seinem Einsatz verworfen werden kann, ohne daß es erforderlich ist, die relativ teuren Saugflaschen einzusetzen und nach ihrem Einsatz zu verwerfen.

Mit der erfindungsgemäßen Anordnung ist es möglich, bei relativ geringem Aufwand eine hohe Infektionssicherheit zu erreichen, wobei gleichzeitig das Krankenhauspersonal hinsichtlich der Wartung der bisher bekannten Einrichtungen entlastet wird.

Ausführungsbeispiele der Erfindung werden nachfolgend anhand der Zeichnungen erläutert. In den Zeichnungen zeigt

Fig. 1    eine erste Ausführungsform der Erfindung, beispielsweise im Einsatzfall als Operationssauger,

Fig. 2    in größerem Maßstab die Ausbildung des Verschlusses für den eigentlichen Saugbehälter in Verbindung mit der besonderen Ausbildung des Auffangbeutels in diesem Bereich und in

Fig. 3    eine Saugflasche, die in Abänderung gemäß der Erfindung für den erfindungsgemäßen Einsatzfall gestaltet ist.

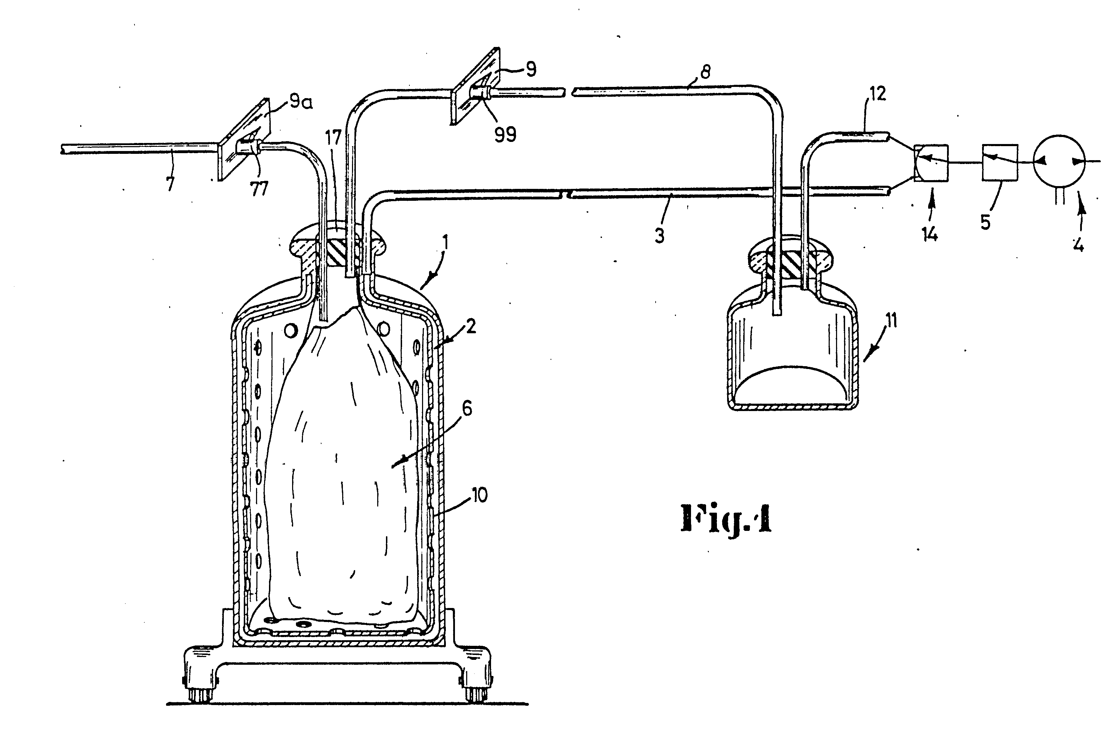

In Fig. 1 ist mit 1 ein Saugbehälter bezeichnet, der beispielsweise aus Glas oder Kunststoff besteht und relativ groß ausgebildet ist. Innerhalb des Saugbehälters 1 ist eine luftdurchlässige Stützwandung 2 angeordnet, wobei zwischen der Stützwandung 2 und der Wandung des Saugbehälters 1 ein Raum geschaffen wird, in dem ein Vakuum oder ein Überdruck erzeugt werden kann.

In diesen Raum mündet zu diesem Zweck eine Saug- und Druckleitung 3, die zu einer vakuum- oder druckerzeugenden Pumpe 4 führt. Die Saug- und Druckleitung 3 kann hierbei als einzelne Leitung oder als zwei getrennte Leitungen gestaltet sein. Bei 5 ist eine Verschlußvorrichtung für die Leitung 3 dargestellt, durch die die Leitung an das System angeschlossen oder von dem System abgekoppelt werden kann bzw. durch die es möglich ist, in der Leitung 3 und in dem Raum zwischen dem Saugbehälter 1 und der Stützwandung 2 entweder einen Überdruck oder einen Unterdruck aufrechtzuerhalten.

Innerhalb des Saugbehälters 1 ist ein Auffangbeutel 6 vorgesehen, der aus Folienmaterial besteht und dadurch durch die relativ kleine Öffnung des Saugbehälters 1 eingeführt werden kann. In den Auffangbeutel 6 mündet eine Saugleitung 7, die zum Operationsfeld zu einer leerzusaugenden Einrichtung oder dergleichen führt. Weiterhin mündet in den Auffangbeutel 6 ein Saugschlauch 8, der zur Pumpe 4 führt.

Der Saugschlauch 8 ist mit einem Verschlußelement, beispielsweise einem Klemmschieber 9, versehen, wobei in diesem Bereich der Saugschlauch 8 durch ein beispielsweise aus Gummi bestehendes, den Klemmschieber 9 tragendes Verbindungsstück 99 unterbrochen ist. Durch den Klemmschieber 9 ist es möglich, den Saugschlauch 8 und dadurch das Innere des Auffangbeutels 6 vom System der Pumpe abzutrennen.

Die Stützwandung 2 des Saugbehälters 1 ist bei dem dargestellten Ausführungsbeispiel als Lochwandung 10 ausgebildet, so daß dadurch die Möglichkeit gegeben wird, bei Erzeugen eines Vakuums im Raum zwischen dem Saugbehälter 1 und der Stützwandung 2 den Auffangbeutel 6 an diese Wandung innen anzulegen, ohne daß ein Durchtritt der Folie des Auffangbeutels 6 durch die Löcher der Lochwandung 10 erfolgt.

Selbstverständlich kann die Stützwandung 2 auch in grundsätzlich anderer Weise ausgebildet sein, beispielsweise relativ vollwandig gestaltet sein und nur wenige Öffnungen zur Einwirkung des Vakuums aufweisen.

Mit 11 ist in Fig. 1 ein Sicherheitsbehälter bezeichnet, in dem der Saugschlauch 8 mündet und aus dem ein Saugschlauch 12 zur Pumpe 4 führt, wobei in diese Verbindung ein Druckreduzierventil 14 eingeschaltet ist, das die Möglichkeit gibt, jeden beliebigen gewünschten Unterdruck im und außerhalb des Auffangbeutels 6 und den erforderlichen Überdruck außerhalb des Auffangbeutels 6 aufrechtzuerhalten. Das Druckreduzierventil 14 arbeitet dabei automatisch derart, daß die jeweils gewünschte Vakuumhöhe im Inneren des Auffangbeutels 6 und in dem Zwischenraum
zwischen Saugbehälter 1 und Stützwandung 2 aufrechterhalten wird, wobei
vorzugsweise das Vakuum im Auffangbeutel 6 niedriger als der Vakuumdruck im Zwischenraum ist.

In die Saugleitung 7 ist ebenfalls eine Schiebeklemme 9a eingesetzt,
die auch von einem den Saugschlauch 7 unterteilenden Gummischlauchstück 77 getragen wird, so daß hier ein Abkoppeln des Systems vom
Saugschlauch 7 ohne Schwierigkeiten möglich ist.

Wie aus Fig. 1 weiterhin ersichtlich, mündet der Saugschlauch 7 in den
Auffangbeutel 6 tiefer als das in den Auffangbeutel 6 reichende Ende
des Saugschlauches 8. Die Länge des in den Auffangbeutel 6 reichenden
Endes des Saugschlauches beträgt dabei höchstens ein Viertel bis ein
Drittel der Länge des Auffangbeutels. Umgekehrt mündet der Saugschlauch
8 in den Sicherheitsbehälter 11 tiefer als der in diesen Sicherheitsbehälter 11 mündende Saugschlauch 12. Durch diese Maßnahme ist sichergestellt, daß ein Mitreißen von aus dem Saugschlauch 7 in den Auffangbeutel 6 und aus dem Saugschlauch 8 in den Sicherheitsbehälter 11 fliessenden Flüssigkeitsmengen durch die zugeordnete zur Pumpe führende Leitung ausgeschlossen ist.

Aus der vorausgehenden Erläuterung und aus der Darstellung ist ersichtlich, daß mit der erfindungsgemäßen Anordnung ein Auffalten des Auffangbeutels 6 innnerhalb des Saugbehälters 1 möglich ist. Weiterhin
kann innerhalb des Auffangbeutels 6 ein Vakuum aufrechterhalten werden, wenn dieses Vakuum kleiner ist als das Vakuum, das zwischen dem
Saugbehälter 1 und der Außenwandung des Auffangbehälters 6 erzeugt wird.

Die Gestaltung des Saugbehälters 1 in Flaschenform, wie in Fig. 1 dargestellt, ist nicht zwingend. Alternativ kann der Saugbehälter 1 beispielsweise als oben offene Tonne ausgeführt sein, wobei der Verschluß
17 dann entsprechend als dicht sitzender Deckel gestaltet ist. Die Saug- und Druckleitung 3 kann außer am Kopf des Saugbehälters 1 auch seitlich am Saugbehälter 1 münden.

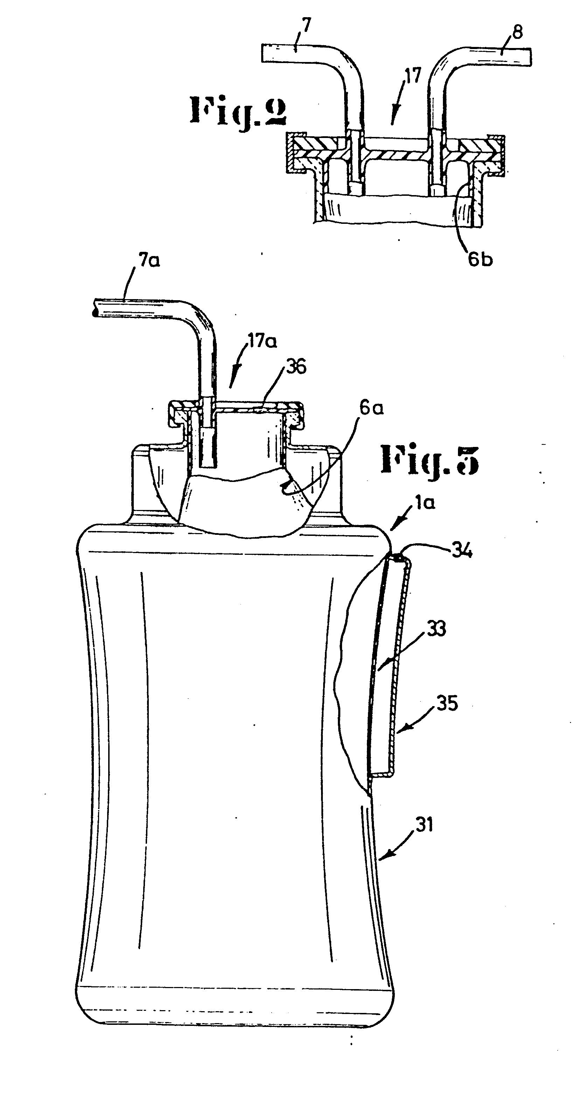

In Fig. 2 ist in etwas größerem Maßstab die Ausbildung des den Saugbehälter abschließenden Verschlusses dargestellt. Der Verschluß 17 ist dabei an dem Saugbehälter 1 festlegbar und verschließt den Saugbehälter 1 vakuumdicht.

Bei 6b ist der obere Bereich des Auffangbeutels 6 erkennbar, der gegenüber dem übrigen Bereich des Auffangbeutels 6 erheblich stärker ausgebildet ist und damit ein Kollabieren in diesem Bereich verhindert. Ausserdem trägt der obere Bereich 6b des Auffangbeutels 6 die Anschlußstücke der Saugleitung 7 und der Saugleitung 8 fest, so daß bei einem Verwerfen des Beutels gleichzeitig diese kurzen Anschlußstücke mit verworfen werden können.

Durch Aufbringen des Verschlusses 17 auf den Saugbehälter 1 erfolgt somit gleichzeitig ein Festlegen und vakuumdichtes Verschließen des Saugbehälters und des Auffangbeutels.

In Fig. 3 ist eine Saugflasche 31 dargestellt, die den Saugbehälter 1a bildet. In diesem Saugbehälter 1a ist ein Auffangbeutel 6a angeordnet, wobei im wesentlichen die gleiche Verschlußanordnung, wie sie in Fig. 2 dargestellt ist, eingesetzt werden kann. Anstelle des bei dieser Einrichtung nicht erforderlichen Saugschlauches 8 ist ein Punktierzentrum 36 im Verschluß 17a vorgesehen.

An der Außenwand der Saugflasche 31 ist eine Ausbuchtung 35 vorgesehen, die gegenüber dem Inneren der Saugflasche 31 durch eine Stützwand 33 abgetrennt wird. Diese Ausbuchtung 35 weist an ihrem oberen Ende ein Punktierzentrum 34 auf, durch das beispielsweise eine Kanüle eingesetzt werden kann, die durch Erzeugen eines Vakuums nunmehr den innerhalb des Saugbehälters 1a angebrachten Auffangbeutels 6a aufbläht, wobei durch den Aufblähvorgang gleichzeitig im Inneren des Auffangbeutels der erforderliche Unterdruck erzeugt wird, der nunmehr über die Saugleitung 7a zum Bereich führt, in dem beispielsweise Wundsekret abgesaugt werden soll.

Wenn die in Fig. 3 dargestellte Saugflasche für die Wunddrainage eingesetzt werden soll, übernimmt die Ausbuchtung 35 und damit das Punktierzentrum 34 die Funktion der Leitung 3 gemäß Fig. 1. Eventuell kann im Bereich des Punktierzentrums 34 auch jede andere beliebige, unterdruckerzeugende Vorrichtung angeschlossen werden, da dieser Bereich nicht steril gehalten werden muß.

Das Sekret wird im Auffangbeutel 6a in der tragbaren, beispielsweise 800 ml fassenden Saugflasche 31 über einen in diese Flasche dicht verschließend eingesetzten Auffangbeutel 6a aufgefangen, wobei vorzugsweise dieser Auffangbeutel 6a ebenfalls das Punktierzentrum 36 aufweist, das anstelle des Leitungsanschlusses 8 gemäß der Ausführungsform entsprechend Fig. 1 tritt. Hier kann eine Probeentnahme und -entleerung während des Drainagebetriebes erfolgen.

Diese Ausführungsform hat gegenüber den bisher bekannten Ausführungsformen von Saugflaschen den Vorteil, daß ein erheblicher Kostenanteil gespart wird und gleichzeitig die Druckregulierung jetzt außerhalb des Auffangbeutels 6a über eine Unterdruckanschlußstelle oder ein Punktierzentrum erfolgen kann.

Patentansprüche

1.      Sauggerät zum Sammeln und Entsorgen von Sekreten und anderen
medizinischen Flüssigkeiten umfassend einen Saugbehälter (1, 1a),
einen in den Saugbehälter (1, 1a) einhängbaren flexiblen Auffangbeutel
(6, 6a), vorzugsweise einen Folienbeutel, der an seiner Einfüllöffnung
abdichtend und fest im Saugbehälter (1, 1a) gehalten ist, wobei der
Auffangbeutel (6, 6a) einen Anschluß zu einer Vakuumquelle und einen
Anschluß für eine Absaugleitung aufweist, eine  unabhängig vom Auffangbeutel (6, 6a) zwischen der Innenseite des Saugbehälters (1, 1a) und
der Außenseite des Auffangbeutels (6, 6a) vorgesehene Anschlußöffnung
für eine zusätzliche Saugleitung (3), und einen auf die Außenseite
des Auffangbeutels (6, 6a) wirkenden Druckanschluß.

2.      Sauggerät nach Anspruch 1, dadurch gekennzeichnet, daß die
Saugleitung (3) auch als Druckleitung betrieben werden kann (Fig. 1
und 3).

3.      Sauggerät nach Anspruch 1 und 2, dadurch gekennzeichnet, daß
der Saugbehälter (1, 1a) im Inneren mit einer Stützwandung (2, 33)
ausgerüstet ist und in den Raum zwischen der Innenseite des Saugbehälters (1, 1a) und der zum Inneren des Saugbehälters (1, 1a) gerichteten
Stützwandung (2, 33) die Saug- und Druckleitung (3) mündet.

4.      Sauggerät nach Anspruch 1 bis 3, dadurch gekennzeichnet, daß
eine handelsübliche tragbare Saugflasche (31) als Saugbehälter (1a) eingesetzt ist, wobei die Saugflasche (31) eine gegenüber dem Inneren der Saugflasche durch eine Stützwandung (33) abgegrenzte, ein Punktierzentrum (34) aufweisende Ausbuchtung (35) besitzt
(Fig. 3).

5.      Sauggerät wenigstens nach Anspruch 1, dadurch gekennzeichnet,
daß die in den Auffangbeutel (6) mündende, einen Unterdruck im Auffangbeutel (6) erzeugende Saugleitung (8) über einen Sicherheitsbehälter (11) zur Vakuumpumpe (4) geführt ist (Fig. 1).

6. Sauggerät wenigstens nach Anspruch 1 und 2, dadurch gekennzeichnet, daß eine vakuum- oder druckerzeugende Pumpe (4) vorgesehen ist und in die Leitungen (3, 8) zwischen der Pumpe (4) und dem Saugbehälter (1) ein Vakuum- bzw. Druckregelventil (14) eingeschaltet ist (Fig. 1).

7. Sauggerät wenigstens nach Anspruch 1, dadurch gekennzeichnet, daß in den Saugschlauch (8) zwischen den Saugbehälter (1) und den Sicherheitsbehälter (11) ein Verschlußelement (9) eingeschaltet ist.

8. · Sauggerät wenigstens nach Anspruch 1, dadurch gekennzeichnet, daß der Saugschlauch (8) vom Saugbehälter (1) zum Sicherheitsbehälter (11) innerhalb des Sicherheitsbehälters (11) tiefer endet als der vom Sicherheitsbehälter (11) zur Pumpe (4) führende Saugschlauch (12) (Fig. 1).

9. Sauggerät nach Anspruch 3, dadurch gekennzeichnet, daß die Stützwand (2) aus einer Lochwand (10) gebildet ist (Fig. 1).

10. Sauggerät nach Anspruch 3, dadurch gekennzeichnet, daß die Stützwand (2) im Saugbehälter (1) vollwandig mit einer oder zwei Durchtrittsöffnungen ausgebildet ist.

11. Sauggerät wenigstens nach Anspruch 1, dadurch gekennzeichnet, daß der den Auffangbeutel (6a) in der Saugflasche (31) festlegende Verschluß (17a) mit einem Sauganschlußstutzen (7a) und einem Punktierzentrum (36) ausgerüstet ist (Fig. 3).

12.     Verfahren zur Aufnahme und Abgabe von medizinischen Sekreten und Flüssigkeiten, wobei als Auffangbehälter für das Sekret oder die Flüssigkeit ein flexibler Auffangbeutel eingesetzt wird, der in einem als Schutzvorrichtung wirkenden, gegenüber der Umgebungsatmosphäre druckdicht abgeschlossenen Saugbehälter angeordnet wird und durch Einwirkung von Unterdruck auf seiner Außenseite innerhalb des Saugbehälters aufgefaltet wird, dadurch gekennzeichnet, daß durch Einwirkung von Überdruck auf die Außenseite des Auffangbeutels dieser wenigstens teilweise entleerbar ist.

13.     Verfahren nach Anspruch 12 mit einem Vakuum- bzw. Druckregelventil, dadurch gekennzeichnet, daß das Vakuum- bzw. Druckregelventil (14) automatisch die Druckverhältnisse innerhalb des Auffangbeutels (6) und außerhalb des Auffangbeutels (6) derart regelt, daß das Vakuum außerhalb des Auffangbeutels (6) größer als das Vakuum im Inneren des Auffangbeutels (6) ist.

**Fig.1**

2/2

0109460

Fig.2

Fig.5

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-4 033 345 (J.L. SORENSON et al.) * Anspruch 4; Figuren 1, 3, 4 * | 1,2,12 | A 61 M 1/00 |
| D,Y | US-A-2 597 715 (E.W. ERIKSON) * Spalte 3, Zeilen 14-18; Figur 3 * | 1,2,12 | |
| D,Y | US-A-3 719 197 (K.A. PANNIER et al.) * Anspruch 1; Spalte 3, Zeilen 5-8; Figur 1 * | 1,2,12 | |
| A | US-A-2 999 500 (F. SCHÜRER) * Spalte 3, Zeilen 16-43; Figur 4 * | 3,9,10 | |
| D,A | DE-B-2 820 517 (STERIMED) * Anspruch 1 * | 4 | **RECHERCHIERTE SACHGEBIETE (Int. Cl. ³)** |
| D,A | DE-C-2 106 631 (C.J. TIMMERMANS) * Spalte 3, Zeilen 43, 44 * | 6 | A 61 J 1/00<br>A 61 M 1/00<br>A 61 M 27/00 |
| A | US-A-3 397 648 (S.A. HENDERSON) * Spalte 4, Zeilen 46-49, 68-70; Figur 6 * | 5,8 | |
| A | US-A-3 875 941 (E.L. ADAIR) * Spalte 3, Zeilen 55-60; Spalte 4, Zeilen 4-7; Figuren 1, 3 * | 7,11 | |

--- -/-

Der vorliegende Recherchenbericht wurde für alle Patentansprüche erstellt.

| Recherchenort BERLIN | Abschlußdatum der Recherche 11-07-1983 | Prüfer CLOT P.F.J. |
|---|---|---|

# EUROPÄISCHER RECHERCHENBERICHT

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 040 427 (HAEMONETICS CORP.) * Seite 4, Zeilen 26, 27; Seite 8, Zeilen 28-35 * | 6,13 | |

-----

RECHERCHIERTE SACHGEBIETE (Int. Cl. 3)

Der vorliegende Recherchenbericht wurde für alle Patentansprüche erstellt.

| Recherchenort BERLIN | Abschlußdatum der Recherche 11-07-1983 | Prüfer CLOT P.F.J. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82